# EUROPEAN PATENT SPECIFICATION

(11) **EP 0 748 616 B1**
(45) Date of publication and mention of the grant of the patent: **09.06.2004**
(21) Application number: 95941895.5
(22) Date of filing: 26.12.1995
(51) Int. Cl.: A61C 8/00

(54) **DENTAL IMPLANT**
ZAHNIMPLANTAT
IMPLANT DENTAIRE

(30) Priority: 28.12.1994 JP 33873194
(43) Date of publication of application: 18.12.1996
(73) Proprietor: Shimoda, Tsunehisa, Fukuoka 814 (JP)
(72) Inventor: Shimoda, Tsunehisa, Fukuoka 814 (JP)
(74) Representative: Modiano, Guido, Dr.-Ing.
(86) International application number: PCT/JP1995/002694
(87) International publication number: WO 1996/019949

(56) References cited:
- WO-A-91/14404
- JP-A- 2 200 258
- JP-A- 5 000 145

## Description

### Technical Field

This Invention relates to the dental implant to be embedded and fixed into mandible or maxilla to fasten such dental prostheses as dentures and cast crown.

### Background Technique:

It is well known that the dental implants having a fixture part to be embedded into bone and an abutment part to be mounted on the tip of said fixture part, which are used to fix the dental prostheses such as dentures have a wide variety of geometrical forms and lengths. These conventional implants have a single structure of insertion into bone and are divided into non-submerged type and submerged type structures by their embedment. The implant for non-submerged type has an abutment exposed in the oral cavity, while that for submerged type which has no such abutment initially will receive an abutment mounted after a certain period from the operation.

The conventional implants required substantial amount of bone to inlay them in. If there is no sufficient amount of bone to embed the implants in, for instance, in such vertical bony defects as upper and lower alveolar bones that have become atrophied by age, the patients were compelled to give up the dental therapy with implants unless the required amount of bone is transplanted.

Other techniques already known in the field of dental implants were described in WO 91/14404, where an implant was described comprising a calotte-like element to be positioned in a fixed manner on the bone to be regenerated. Such calotte-like implant needed to have a particular structure, with shaped perforations on its surface to permit rapid and adequate revascularization of the blood clot. Moreover, a certain rigidity of the structure was also desirable to obtain a stable structure. Although the above solution had some degree of effectiveness, it needed the manufacture of the complex calotte-like element, which involved noticeable manufacturing costs.

On the other hand, the Distraction Osteogenesis Theory first described in 1988 by G. A. Ilizarov, a Russian medical doctor has attracted public attention as a medical method applicable to the treatment of malformation or such therapy against abnormal bone length. This osteogenetic method has produced good results over 35 years in correction of the form of the bone and soft tissue. Though this technique has not so far been applied to any vertical bone augmentation, it is considered to be applicable not merely to orthopedic field but also to stomatognathic field, for example, to the improvement of the length, width and radius of the lower jaw. This method has been experimentally studied also in Japan.

The idea of augmented amount of bone to recover the bony form against substantial bone defect, malformation and atrophy has great medical possibilities. That is, this idea is based on the unique Distraction Osteogenesis Theory, which is in its turn based on the concept that the continuous stimulating force has osteogenetic power and capacity of mucous membrane expansion.

On the other hand, such an idea as osteogenesis have never been conceived up to now in any clinical studies and researches of dental implants. The implant body has thus far been embedded only into a given bone.

### Disclosure of the Invention:

The purpose of this invention is to provide a dental implant that can be firmly fixed into oral cavity securing or producing the amount of bone required for the embedment of the implant by applying the idea of said distraction osteogenesis even without performing a bone transplantation.

To attain the above objective this invention has been of such constitution as follows. That is, the dental implant by this invention is characterized in that the fixture part to be fastened into bone consists of plural members so connected as adjustable into their effective length. More materially it is preferred that a rod-shaped, tapped middle member which has a male screw be screwed and incorporated into a cylindrical threaded exterior member which has a female screw.

The fixture part of the implant by this invention is embedded into maxilla or mandible, and its length is extended at suitable period to lift up the mucous membrane on bone surface into a tent-like shape thereby making act the continuous stimulating force having osteogenetic power or mucous membrane augmenting capacity to guide physiologically the bone to the circumference of the implant in the cavity that has produced because the mucous membrane was lifted up. Required amount of bone is thus produced around the implant without any particular bone transplantation to hold firmly the implant. This invention allows us to produce required amount of bone around the implant to hold said implant firmly making use of the unique idea of the distraction osteogenesis based on the concept that the continuous stimulating force has osteogenetic power and mucous membrane expanding capacity. The implant according to this invention is therefore considered to be a landmark implant which is conceptually quite different from the conventional ones.

### Brief Description of Drawings:

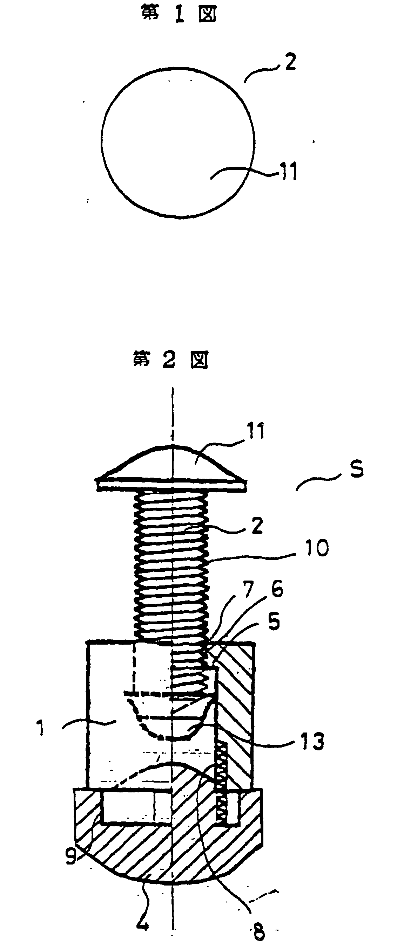

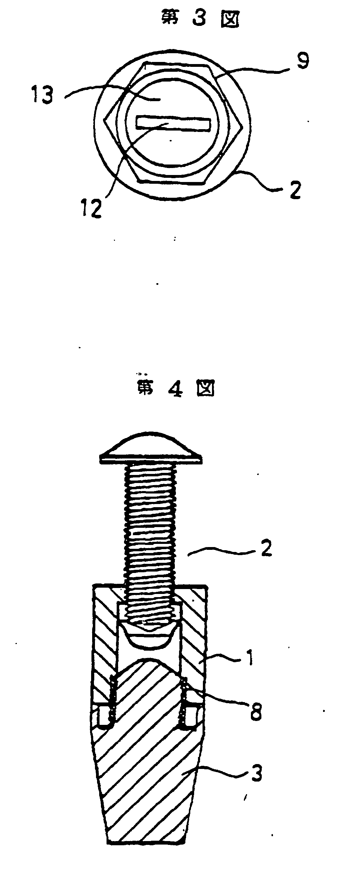

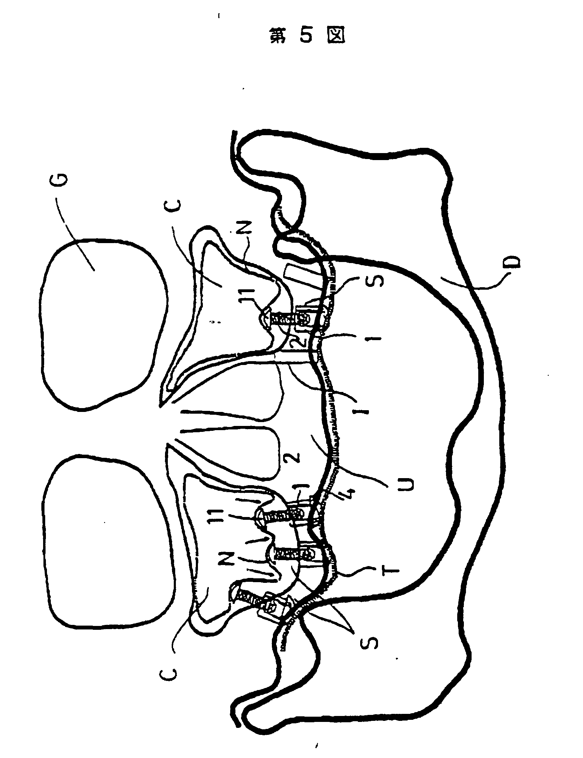

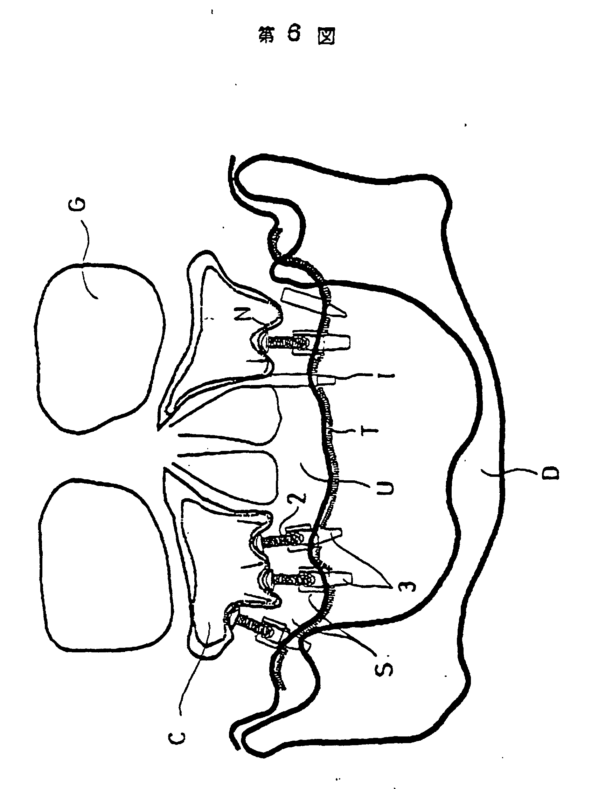

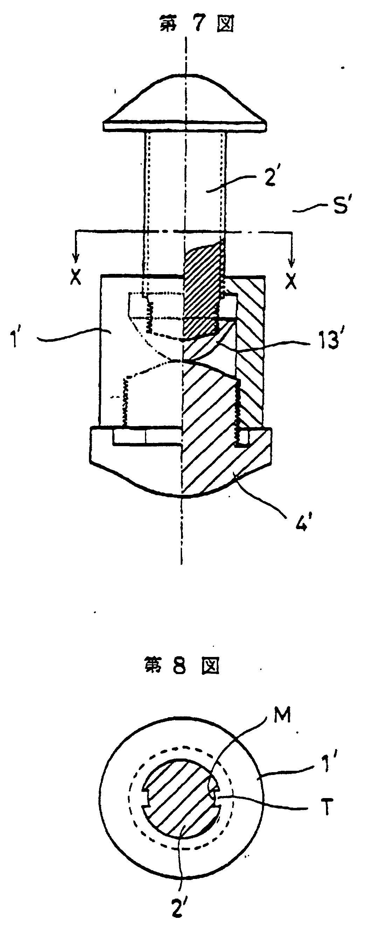

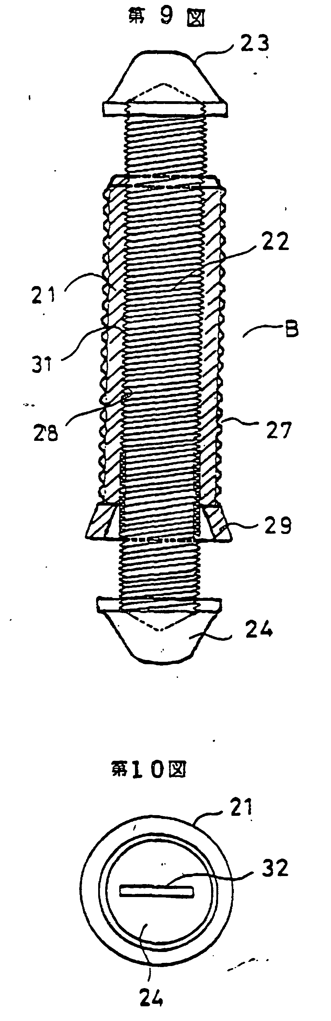

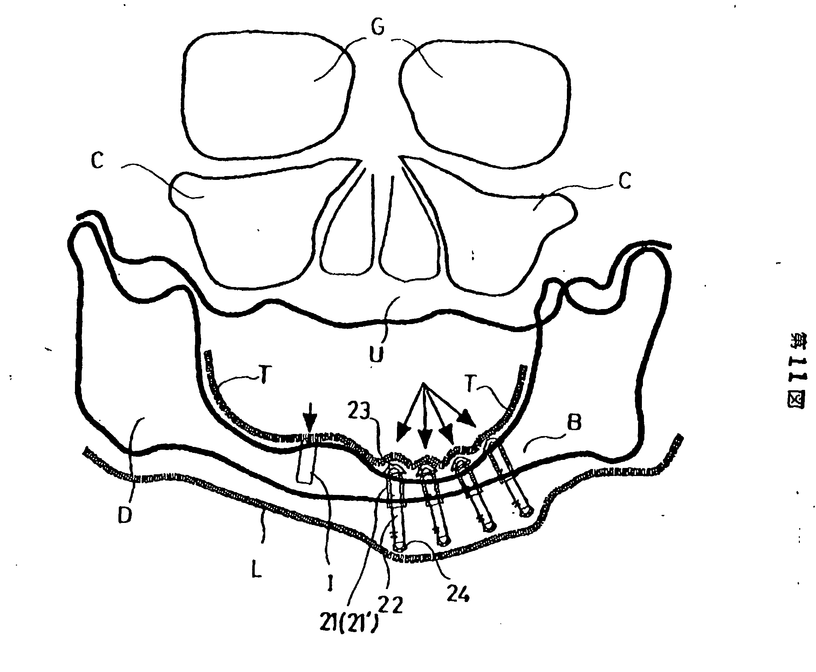

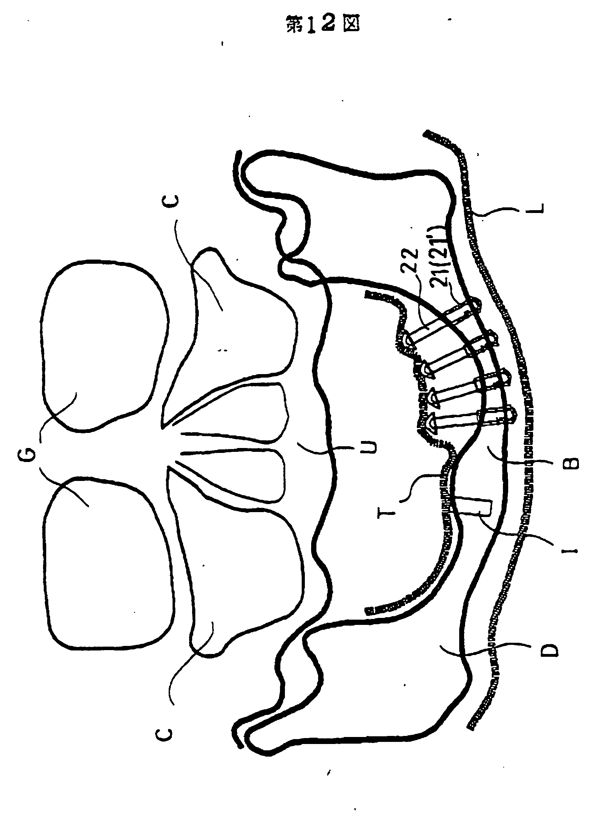

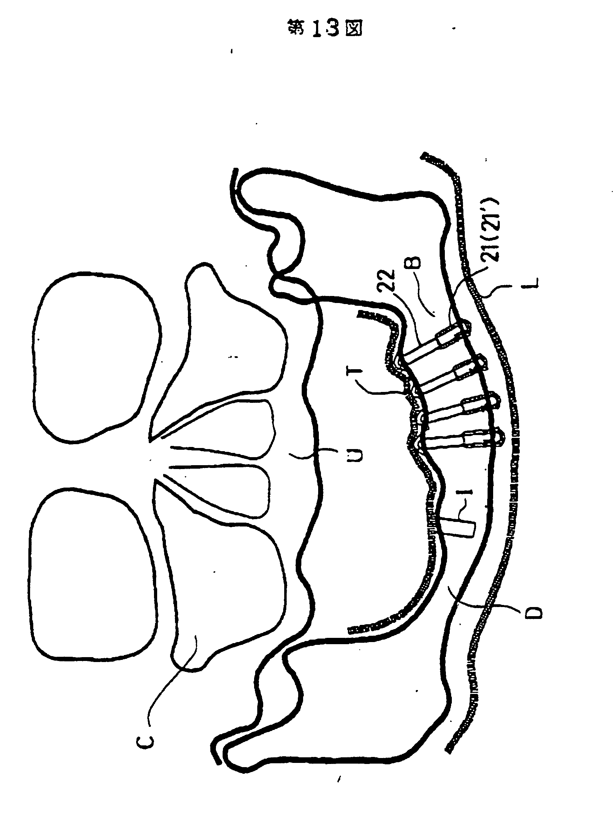

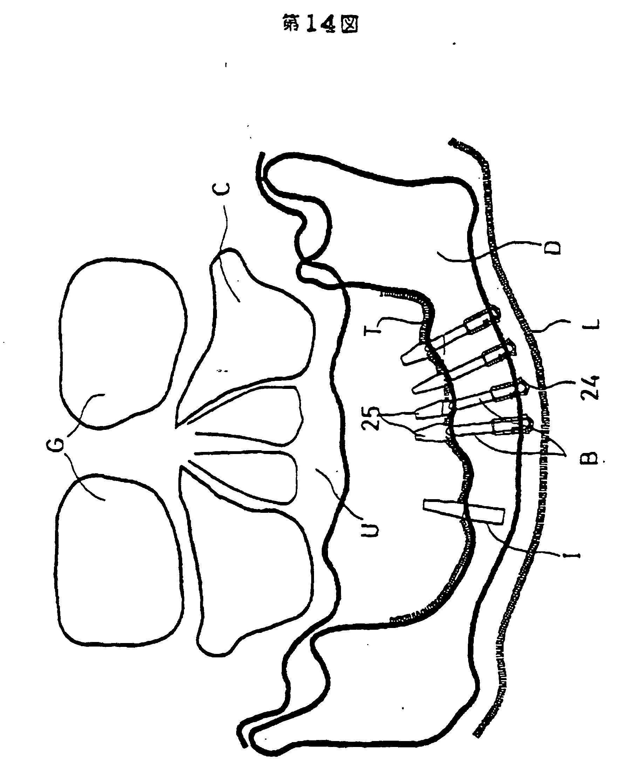

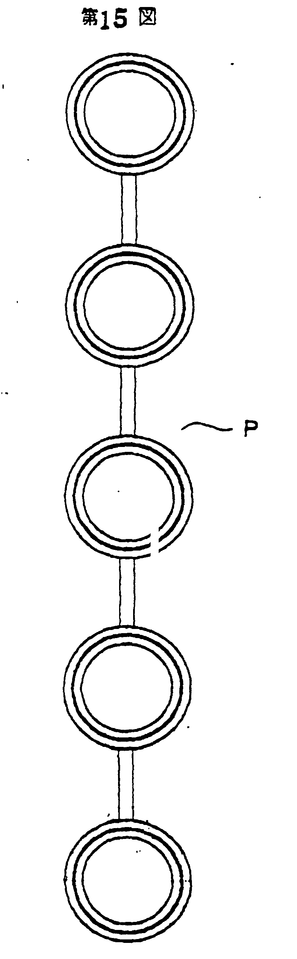

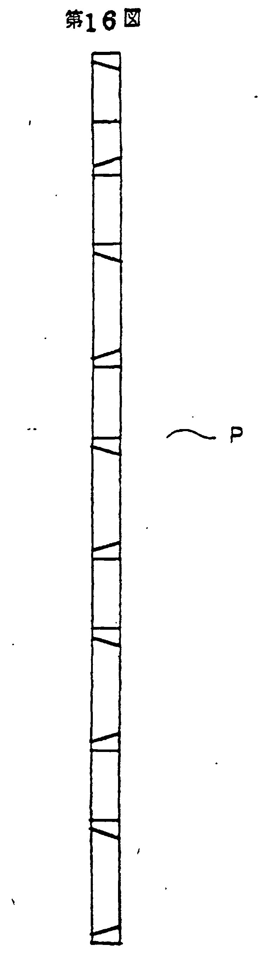

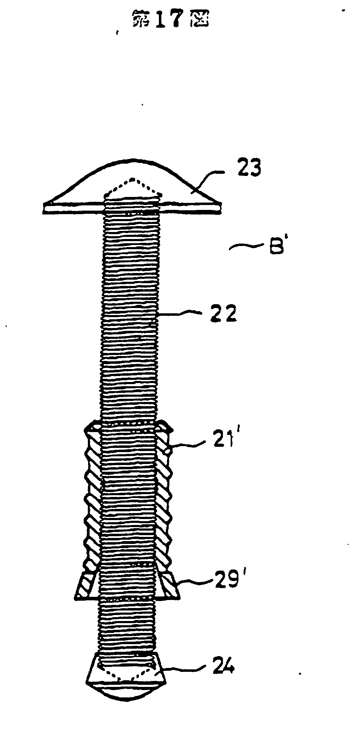

Fig. 1 is a plan view of the middle member. Fig. 2 is a partial (right half) longitudinal sectional view of the implant for upper jaw where the middle member and a healing cap are screwed into the exterior member. Fig. 3 represents a bottom view of the same implant where the healing cap has been removed. Fig. 4 is another longitudinal sectional view of the same implant with an abutment mounted. Fig. 5 is an explanatory drawing of the implant for upper jaw just after the operation of embedment into the maxilla. Fig. 6 is another explanatory drawing that illustrates the same implant six months after the operation. Fig. 7 is a vertical sectional view of an implant for upper jaw which is different from the foregoing one. Fig. 8 is an X-X sectional view of the same implant. Fig. 9 is a vertical sectional view showing how the fixture part is connected in the implant for lower jaw, and Fig. 10 represents a bottom view of this implant. Fig. 11 is an exemplary drawing of an implant for lower jaw just after the operation of its embedment into mandible. Figs. 12 and 13 represent the explanatory drawings of the same implant after one month and six months from the operation, respectively. Fig. 14 is another explanatory drawing of the same implant, but with abutment mounted. Fig. 1 5 is a front elevation of an exterior member connection plate, and Fig. 16, a side view of the same plate. Fig. 17 is a vertical sectional view of an implant for lower jaw in a different embodiment.

### Best Form for Working of the Invention:

In the exemplary embodiments of the invention as disclosed in the drawings, Fig. 1 through Fig. 4 represents an implant for upper jaw S as an embodiment of this invention. The implant for upper jaw S has the exterior member 1 and the middle member 2 which are screwed into each other, the abutment 3 which can be mounted on the lower edge of the exterior member, and the healing cap 4.

The exterior member 1 is formed into a cylinder that opens at its lower edge and provided, at its upper edge, with the inward flange 5, at the center of which is provided a tapped hole 7 with female screw 6. The lower inner circumference of the exterior member 1 is provided with a female screw portion 8 for screwing the healing cap 4 or abutment 3. The lower edge of the exterior member 1 has a hexagonal nut 9.

The middle member 2 has an uppermost member 11 at the top of the rod-like body on the outer circumference of which is threaded a male screw 10, and incorporates at its lower edge an edge member 13 having a groove 12 for engagement with a flat or cruciform screwdriver.

As shown in Fig. 5, the exterior member 1 of the implant for upper jaw S is inlaid into a human,maxilla U from the oral cavity side, and the middle member 2 screwed into this exterior member is turned by a screwdriver so that the uppermost portion thereof is projected upward. The healing cap 4 has been screwed into the lowermost portion of the exterior member 1. Thus, the uppermost portion of the middle member protrudes into the maxillary sinus C to lift up the mucous membrane N with its uppermost member 11 thereby producing a space or cavity between the mucous membrane and the maxilla U. Letter I in this figure represents a conventional implant, D mandible, and G the eyeballs. T represents the mucous membrane on the inner face of the oral cavity. At the operation, this mucous membrane is incised to embed the implant.

If this state is left to stand for a suitable period (six months, for example), bone is formed in the cavity under the mucous membrane N as lifted into the interior of the maxillary sinus C with the middle member 2 of the implant embedded into this bone fastening thus the implant S as a whole firmly. When this state comes, the abutment 3 is screwed into the screw portion in the opening of the exterior member 1 so that it is exposed to the outside of the mucous membrane T in the oral cavity. Such prostheses as dentures can be mounted by way of this abutment. One may adopt welding as a mounting method for this abutment.

Though this implant S belongs to the implant for submerged type by conventional classification, it is pathophysiologically and structurally different from the conventional implants, which seek after the construction of the implant body as developed based on its maintenance in the bone and after the bone-affinity type strength of its materials. That is, the implant by this invention has been so designed that the intrinsic maxillary sinus in maxilla with relatively small amount of bone is narrowed to induce the production of the bone, based on the original ideal of the distraction osteogenesis, which in its turn is based on the concept that the continuous stimulating force has bone producing power and mucous membrane augmenting capacity.

This design with a unique double structure screw allows theoretically to extend the effective length of the implant itself about twice the initially embedded implant. This implant features further such an excellent characteristic that the middle member which can lift up the mucous membrane of maxillary sinus may introduce bone around itself. From structural point of view, the middle member plays a role of anchor in the bone. Moreover, this implant displays an excellent holding power, since it has a bicortical structure in which both the maxillary sinus and oral cavity sides are surrounded by the cortical bone.

Figs. 7 and 8 illustrate an embodiment of an implant for maxilla, different from the foregoing implant, in which the exterior member 1' and middle member 2' of the implant S' are slidably connected. Provided symmetrically on the lateral side of the middle member 2' are two grooves M and M all along the axis with which slidably engaged are the protrusions T and T provided on the inward flange F of the exterior member 1'. Screwed at the extremity of the middle member 2' is the end member 13' which is attached, for positioning, to the healing cap 4' screwed at the extremity of the exterior member 1'. The projected length of the middle member can be freely adjusted by changing the screwed depth of the healing cap 4' as against the exterior member. At this adjustment, the middle member slides on the exterior member. It should be understood that this invention is not limited to these embodiments but that the connected length of the middle and exterior members have only to be adjustable.

Figs. 9 and 10 represent an embodiment of the implant for mandible B, which have the exterior member 21, middle member 22, healing cap 23 and end member 24. The exterior member 21 is a cylinder open in its upper and lower portions, on the outer and inner circumferences of which are provided respectively rough-pitched and fine-pitched male and female screws 27 and 28. The lowermost portion of the exterior member 21 is provided with flared guide 29.

The middle member 22 is formed into a rod, provided on the outer circumference of which is a male thread 31 screwed into the female screw 28 of said exterior member. Healing cap 23 and end member 24 are screwed into the uppermost and lowermost portions of said middle member 22, respectively. The geometrical form of the healing cap 23 is similar to one in said implant S. A groove (flat or cruciform) for engagement with a screwdriver is formed on the end member 24. Fig. 17 represents an implant for mandible B' having a little different exterior member 21'.

The implant B embeds its exterior member 21 (21') screwed into the middle member 22 into jaw bone D. For this, the middle member 22 has been screwed into the exterior member 21 (21') to the extent that the healing cap 23 comes closest to the uppermost portion of the exterior member 21 (21'). Just after this embedment, the healing cap 23 holds up more or less the mucous membrane T in the oral cavity, while the end member 24 mounted on the lowermost portion of the middle member holds down the skin L under the jaw. The middle member thus positioned just under the skin can be palpated. The implant will be observed in this state for a suitable period, for example, for two weeks.

After said observation period has elapsed, the middle member 22 is screwed into daily little by little, for example, by 2 mm each. Fig. 1 2 depicts the state of the middle member one month after the operation. In about two weeks, the middle member 22 is pierced through into the oral cavity from just under the skin to expand the mucous membrane T of the oral cavity. The implant is observed in this state for a suitable period, for about 6 months.

Fig. 13 represents the state 6 months after the operation where newly generated bone is observed around the implant B.

Fig. 1 4 illustrates the implant B firmly held by the bone generated around said implant, where the healing cap has been undone and the abutment 25 is mounted to protrude into the oral cavity outside the mucous membrane T. Dentures, cast crown and the like are to be mounted on this abutment.

The implant B for lower jaw needs only manipulation from inside the oral cavity at the initial operation without any incision over the skin. Even the secondary operation requires only an extremely small skin incision just for the turns of screw intended to apply the continuous stimulating force. As was the case with said implant for upper jaw S, the middle member plays a role of anchor in the bone and can have a bicortical structure surrounded by the cortical bone, displaying thus an excellent holding power. Though the figure shows the osteogenesis in the oral cavity, the bone may be generated on the submaxillary edge by inlaying the implant invertedly.

In addition to the implant for lower jaw to be embedded from inside the oral cavity, there is another type of implant that necessitates skin incision from the submaxillary edge. We can use, for this latter type, a plate P (Symphysis Plate) that can join respective exterior members as shown in Figs. 15 and 1 6.

As this invention may be embodied in several geometrical forms and dimensions without departing from the scope of the claims, the present embodiments are therefore illustrative and not restrictive. Though said figures show the external and middle members connected with screws so that their length may be variable, it is possible to adopt other types of structure that allow to adjust their length adequately. It is also possible to adopt triple or higher construction in place of the illustrated double structure of external and middle members.

The material of the implant by this invention may be some material which is not harmful to human body and excellent in strength, for example, titanium or titanium alloy which is conventionally known implant material.

### Industrial Availability:

The dental implant according to the present invention may be industrially manufactured from titanium, titanium alloy or other metals by conventionally known processing technique to be used as a fixing member for dental prostheses.

## Claims

1. A dental implant comprising fixture parts to be embedded into the maxilla or upper jaw bone, said implant comprising a plurality of members, including a threaded exterior member (1) and a threaded internal member (2) screwable one with respect to the other, forming a combined implant,
**characterized in that** said implant is variably adjustable in its length for lifting a mucous membrane (T), the length adjustment being performed by acting on one of said plurality of members (2) which is on the side of the maxilla in which the implant is inserted, i.e. on the oral side of the upper jaw, and the lifting of the mucous membrane (T) occurring on the opposite side of the maxilla in which the implant is inserted, i.e. in the maxillary sinus cavity (C).

2. A dental implant comprising fixture parts to be embedded into the mandible or lower jaw bone, said implant comprising a plurality of members, including a threaded exterior member (21, 21') and a threaded internal member (22, 22') screwable one with respect to the other forming a combined implant,
**characterized in that** said implant is variably adjustable in its length for lifting a mucous membrane (T), the length adjustment being performed by acting on one of said plurality of members (22, 22") which is on the opposite side of the side of the mandible in which the implant is inserted, i.e. on the lower part of the mandible, and the lifting of the mucous membrane (T) occurring on the opposite side of the mandible in which the implant is inserted, i.e. in the oral cavity.

3. A dental implant as claimed in claim 2, **characterized in that** it comprises a symphysis plate (P) joining respective exterior members (21, 21') and necessitates skin incision from the submaxillary edge.

4. The dental implant as claimed in claim 1, 2 or 3, **characterized in that** said fixture parts are provided with a cylindrical external member (1, 21, 21') on the inner circumference of which a female screw is tapped and a rod-shaped middle member (2, 22, 22') which is threaded into said female screw of said external member and that said external and middle members are screwed into each other in one block so that their length is adjustable.

5. The dental implant as claimed in claim 1, 2 or 3, **characterized in that** said fixture parts are so constructed that the plurality of members are connected in such a fashion that may be axially slidable on each other.

## Patentansprüche

1. Ein Zahnimplantat, das Befestigungsteile umfasst, die in die Maxilla oder den Oberkieferknochen einzubetten sind, wobei das Implantat eine Vielzahl von Elementen umfasst, einschließlich eines Außenelements (1) mit Gewinde und eines Innenelements (2) mit Gewinde, die in Bezug zueinander zusammenschraubbar sind und somit ein kombiniertes Implantat bilden,
**dadurch gekennzeichnet, dass** das Implantat in seiner Länge variabel anpassbar zum Anheben einer Schleimhaut (T) ist, wobei die Längenanpassung durchgeführt wird durch Einwirken auf eines der Vielzahl von Elementen (2), das sich auf der Seite der Maxilla befindet, in die das Implantat eingesetzt wird, d. h. auf der oralen Seite des Oberkiefers, und wobei das Anheben der Schleimhaut (T) auf der gegenüberliegenden Seite der Maxilla stattfindet, in die das Implantat eingesetzt wird, d. h. in die Nebenhöhle (C) der Maxilla.

2. Ein Zahnimplantat, das Befestigungsteile umfasst, die in die Mandibula oder den Unterkieferknochen einzubetten sind, wobei das Implantat eine Vielzahl von Elementen umfasst, einschließlich eines Außenelements (21, 21') mit Gewinde und eines Innenelements (22, 22') mit Gewinde, die in Bezug zueinander zusammenschraubbar sind und so ein kombiniertes Implantat bilden,
**dadurch gekennzeichnet, dass** das Implantat in seiner Länge variabel anpassbar zum Anheben einer Schleimhaut (T) ist, wobei die Längenanpassung durchgeführt wird durch Einwirken auf eines der Vielzahl von Elementen (22, 22"), das sich auf der gegenüberliegenden Seite der Seite der Mandibula befindet, in welche das Implantat eingesetzt wird, d. h. im unteren Teil der Mandibula, und wobei das Anheben der Schleimhaut (T) auf der gegenüberliegenden Seite der Mandibula stattfindet, in die das Implantat eingesetzt wird, d. h. in die Mundhöhle.

3. Ein Zahnimplantat wie in Anspruch 2 beansprucht, **dadurch gekennzeichnet, dass** es eine Symphysenplatte (P) umfasst, welche die jeweiligen Außenelemente (21, 21') verbindet und einen Hauteinschnitt von der submaxillaren Kante aus erforderlich macht.

4. Das Zahnimplantat wie in Anspruch 1, 2 oder 3 beansprucht, **dadurch gekennzeichnet, dass** die Befestigungsteile mit einem zylindrischen Außenelement (1, 21, 21') ausgestattet sind, an dessen Innenumfang eine Schraubenmutter angebracht wird, und mit einem stabförmigen mittleren Element (2, 22, 22'), das in die Schraubenmutter des Außenelements eingeschraubt wird, und dadurch, dass die äußeren und mittleren Elemente in einem Block ineinander eingeschraubt werden, so dass ihre Länge verstellbar ist.

5. Das Zahnimplantat wie in Anspruch 1, 2 oder 3 beansprucht, **dadurch gekennzeichnet, dass** die Befestigungsteile so konstruiert sind, dass die Vielzahl von Elementen so verbunden werden, dass sie axial aufeinander verschoben werden können.

## Revendications

1. Un implant dentaire comprenant des parties de fixation à incorporer dans l'os du maxillaire ou mâchoire supérieure, ledit implant comprenant une pluralité d'éléments, incluant un élément à filetage extérieur (1) et un élément à filetage intérieur (2) susceptibles d'être vissés l'un sur l'autre, en formant un implant combiné,
**caractérisé en ce que** ledit implant est ajustable de façon variable en longueur, pour lever une membrane muqueuse (T), l'ajustement en longueur étant effectué en agissant sur l'un de ladite pluralité d'éléments (2) se trouvant sur le côté de la maxillaire dans lequel l'implant est inséré, c'est-à-dire sur le côté oral de la mâchoire supérieure, et la levée de la membrane muqueuse (T) se produisant sur le côté opposé de la maxillaire dans laquelle l'implant est inséré, c'est-à-dire dans la cavité sinusale maxillaire (C).

2. Un implant dentaire comprenant des parties de fixation à incorporer dans l'os de la mandibule ou mâchoire inférieure, ledit implant comprenant une pluralité d'éléments, comprenant un élément à filetage extérieur (21, 21') et un élément à filetage intérieur (22, 22'), susceptibles d'être vissés l'un sur l'autre en formant un implant combiné,
**caractérisé en ce que** ledit implant est ajustable de façon variable en longueur, pour lever une membrane muqueuse (T), l'ajustement en longueur étant effectué en agissant sur l'un de ladite pluralité d'éléments (22, 22") qui se trouve sur le côté opposé au côté de la mâchoire inférieure dans lequel l'implant est inséré, c'est-à-dire sur la partie inférieure de la mâchoire inférieure, et la levée de la membrane muqueuse (T) se produisant sur le côté opposé de la mâchoire inférieure, dans lequel l'implant est inséré, c'est-à-dire dans la cavité orale.

3. Implant dentaire selon la revendication 2, **caractérisé en ce qu'**il comprend une plaque de symphyse (P), reliant les éléments extérieurs (21, 21') respectifs et nécessisant une incision de la peau depuis le bord submaxillaire.

4. L'implant dentaire selon la revendication 1, 2 ou 3, **caractérisé en ce que** lesdites parties de fixation sont munies d'un élément externe (1, 21, 21') cylindrique, sur la circonférence intérieure duquel un filetage femelle est taillé, et un élément médian (2, 22, 22') en forme de tige, vissé dans ledit filetage femelle dudit élément externe, et **en ce que** lesdits éléments externes et médians sont vissés l'un dans l'autre pour former un bloc, de manière que leur longueur soit ajustable.

5. L'implant dentaire selon la revendication 1, 2 ou 3, **caractérisé en ce que** lesdites parties de fixation sont construites de manière que la pluralité d'éléments soient reliés de manière à pouvoir être coulissés axialement l'un sur l'autre.
